(19) **Europäisches Patentamt**
**European Patent Office**
**Office européen des brevets**

(11) **EP 3 677 554 B1**

(12) **EUROPÄISCHE PATENTSCHRIFT**

(45) Veröffentlichungstag und Bekanntmachung des Hinweises auf die Patenterteilung:
**29.12.2021   Patentblatt 2021/52**

(51) Int Cl.:
*C03B 23/055* *(2006.01)*    *C03B 23/09* *(2006.01)*

(21) Anmeldenummer: **19197214.0**

(22) Anmeldetag: **13.09.2019**

(54) **VERFAHREN UND VORRICHTUNG ZUR HEISSFORMUNG VON GLÄSERNEN WERKSTÜCKEN UND HEISSUMGEFORMTE GLASBEHÄLTER**

METHOD AND DEVICE FOR THERMOFORMING GLASS WORKPIECES AND THERMOFORMED GLASS CONTAINERS

PROCÉDÉ ET DISPOSITIF DE MOULAGE À CHAUD DES PIÈCES EN VERRE ET RÉCIPIENTS EN VERRE MOULÉS À CHAUD

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.10.2018   DE 102018126053**

(43) Veröffentlichungstag der Anmeldung:
**08.07.2020   Patentblatt 2020/28**

(73) Patentinhaber: **SCHOTT Schweiz AG**
**9001 St. Gallen (CH)**

(72) Erfinder:
• **JUD, Xaver**
**9217 Neukirch a.d. Thur (CH)**

• **LEE, Robert**
**21500 Suzhou City (CN)**
• **JIANG, Sherry**
**Wuzhong District**
**Suzhou, 215100 Jiangsu (CN)**

(74) Vertreter: **Blumbach · Zinngrebe Patentanwälte PartG mbB**
**Alexandrastraße 5**
**65187 Wiesbaden (DE)**

(56) Entgegenhaltungen:
**WO-A1-2018/011148     DE-C- 45 584**
**DE-C- 46 337**

**Beschreibung**

Gebiet der Erfindung

Die Erfindung betrifft ein Verfahren und eine Vorrichtung zum Formen von gläsernen Werkstücken in einem Heißprozess. Im Speziellen betrifft die Erfindung ein Verfahren sowie eine Vorrichtung für einen entsprechenden Umformprozess, welche es ermöglichen, die Produktion von Gutstücken durch vergrößerte Reinigungsintervalle zu steigern. Des Weiteren betrifft die Erfindung hohle Glasgegenstände.

Hintergrund der Erfindung

Aus dem Stand der Technik sind Verfahren bekannt, bei denen Glasgegenstände durch einen Umformprozess aus einem Werkstück, auch als Halbzeug bezeichnet, hergestellt werden. So werden Glasfläschchen, beispielsweise für pharmazeutische Anwendungen aus einem Glasrohr hergestellt, in dem ein kurzes Rohrstück an einem Ende bis zur Formgebungstemperatur des Glases erhitzt wird und mit einem oder mehreren Formgebungsschritten mit geeigneten Formwerkzeugen in die gewünschte Form gebracht werden. Die Innengeometrie wird üblicherweise mit Dornen geformt, die in das Rohrende eingebracht werden. Das Werkstück rotiert während des Formgebungsprozesses. Die Form und Ausmaße des Dorns definieren hierbei die Innengeometrie des Glasfläschchens. Die Umformung erfolgt durch äußere Formwerkzeuge, welche das Glasrohr gegen den Dorn drücken und gleichzeitig die äußere Seite des Glasrohrs ausformen. Hierbei dreht sich das Glasrohr und das äußere Formwerkzeug. Das äußere Formwerkzeug umfasst eine Formgebungsrolle mit einer Formgebungsfläche

So beschreibt die DE 202004004560 U1 ein Verfahren und eine Vorrichtung zur Heißumformung mit frei drehbar gelagerten Formgebungsrollen. Die Formgebungsrollen werden durch das rotierende Werkstück angetrieben und drehen sich synchron mit dem Glas, d.h. es wird zwischen Glas und den Formgebungsrollen keine Relativbewegung durchgeführt.

WO 2018011148 A1 offenbart eine Vorrichtung zur Herstellung eines Werkstücks aus Glas, einschließlich einer Einrichtung zur Erwärmung, ein inneres Formwerkzeug, äußere Formgebungsrollen und eine Vorrichtung zum Aufbringen von Schmieröl auf die Rollen. WO 2018011148 A1 lehrt auch ein entsprechendes Verfahren zur Nutzung dieser Vorrichtung. Es werden Glasfläschchen mit Hals und Schulter hergestellt.

Bevorzugt erfolgt die Umformung an einem sogenannten Rundtakter. Die Umformung kann hierbei in mehreren Schritten, d.h. an mehreren Stationen des Rundläufers erfolgen.

Während des Werkzeugkontakts wird das gläserne Werkstück durch die Formwerkzeuge gekühlt. Zwischen den Formungsschritten muss das Werkstück daher gegebenenfalls wieder erwärmt werden. Die Temperaturführung wird so gestaltet, dass nach dem letzten Formgebungsschritt das Glas eine Temperatur erreicht, bei der das Werkstück formstabil ist.

Durch den Kontakt mit dem heißen Glas sind die Formwerkzeuge, insbesondere die Formgebungsflächen der Formgebungsrolle, hohen Temperaturbelastungen ausgesetzt. Das Glas kann dabei Temperaturen bis zu 1000 °C aufweisen. Während des Umformprozesses erhitzen sich somit die Formgebungsflächen und können so Temperaturen von > 250 °C aufweisen.

Zudem muss ein direkter Kontakt der Formwerkzeuge mit dem heißen Glas vermieden werden, da dies zu einem Anhaften des Glases auf der Oberfläche der Formwerkzeuge führt. Daher wird bei der oben beschriebenen Heißumformung von Glas in der Regel ein Schmiermittel, auch als Trennmittel bezeichnet, beispielsweise ein Öl oder eine Paste, verwendet. Hierbei wird das Schmiermittel in den Zwischentakten des Formprozesses, in denen die Werkzeuge keinen Glaskontakt haben, auf die Formwerkzeuge aufgebracht, beispielsweise durch ein Besprühen, Anspritzen, Aufspritzen oder Überspülen der Formwerkzeuge mit dem Schmiermittel. Hierbei wird das jeweilige Werkzeug vor jedem Glaskontakt erneut geschmiert.

Auf Grund der hohen Temperaturen entstehen während des Umformprozesses Reaktionsprodukte der Schmiermittel. So kommt es zur Rußbildung. Während des Betriebs werden somit auf den Formgebungsflächen der Formgebungsrolle Rußablagerungen aufgebaut. Dies ist problematisch, da der Ruß in Kontakt mit dem heißen Glas kommt und sich so in das formbare Glas einarbeiten bzw. mit dem Glas verbinden kann. Dies verursacht erhebliche kosmetische Probleme. Bei der Herstellung von Glasfläschchen führt dies beispielsweise zu einer Kontamination im Bereich des umgeformten Flaschenhalses. Die entsprechenden Glasfläschchen müssen ausgeschieden werden.

Neben einer Rußkontamination können die Konturen von unebenen Rußablagerungen auf den Formrollen auf das Glas übertragen werden. Die Unebenheiten werden dabei wie bei einem Stempel auf die Glasoberfläche aufgedrückt, was ebenfalls zu kosmetischen Defekten und somit zu einem Ausschuss der entsprechenden Fläschchen führt. Des Weiteren kann es auch zu Einschlüssen des Schmiermittels zwischen Glasoberfläche und den rotierenden Formrollen kommen. Der eingeschlossene Ölfilm wird während des Abrollvorgangs zwischen Glas- und Werkzeugoberfläche eingeklemmt und prägt somit ein Muster auf die noch verformbare Glasoberfläche. Dies führt beim Endprodukt zu einer fleckenartigen Struktur mit abweichenden Höhen auf den entsprechenden Glasoberflächen. Hierbei besteht kein grundsätzlicher Unterschied in der Ausbildung des Musters bezüglich der Richtung, d.h. es besteht kein grundsätzlicher Unterschied zwischen der Ausprägung des Musters in der Umlaufrichtung, d.h. tangential, und der Ausprägung des Musters in Richtung der Rotationsachse, also axial.

Daher müssen die Formwerkzeuge in einem geeigneten Reinigungsintervall gereinigt werden. Typischerweise beträgt dieses Intervall 2 bis 3 Stunden. Hierbei ist zur Reinigung der Formrollen eine Stilllegung der Produktionsmaschine notwendig, was zu einer Minderproduktion führt. Darüber kann es durch die Stilllegung der Produktionsmaschine zu Anlaufproblemen kommen, so dass der Herstellungsprozess über die Stilllegungsdauer hinaus beeinträchtigt wird.

## Aufgabe der Erfindung

Eine Aufgabe der Erfindung liegt darin, eine Vorrichtung zur Herstellung eines Glasgegenstands mit definierter Innen- und Außengeometrie bereit zu stellen, welche die oben beschriebenen Nachteile des Stands der Technik nicht aufweist. Weitere Aufgabe der Erfindung bestehen in der Bereitstellung eines entsprechenden Herstellverfahrens sowie eines hohlen Glasgegenstandes.

## Beschreibung der Erfindung

Die Aufgabe der Erfindung wird bereits durch den Gegenstand der unabhängigen Ansprüche gelöst. Vorteilhafte Ausführungsformen und Weiterbildungen der Erfindung sind Gegenstand der Unteransprüche.

Die erfindungsgemäße Vorrichtung weist hierbei zumindest folgende Komponenten auf:

- Einrichtung zur Erwärmung des Glases bis zur Erweichung,
- Eine Formstation mit zumindest einem inneren und einem äußeren Formwerkzeug zum Formen des Werkstücks wobei das äußere Formwerkzeug eine Formgebungsrolle mit einer Formgebungsfläche umfasst und zur Formung der äußeren Mantelflächen des Werkstücks dient sowie einer Vorrichtung zur Aufnahme der Formgebungsrolle und
- einer Vorrichtung zum Aufbringen eines Schmieröls auf die Formgebungsfläche der Formgebungsrolle mit einer Auslassöffnung zur Abgabe des Schmieröls, wobei

die Formgebungsrolle in der Vorrichtung zur Aufnahme frei drehbar gelagert ist und durch eine verriegelbare Arretierungseinrichtung während des Formgebungsprozesses fest fixierbar bzw. fixiert ist.

Die Ausformung des Werkstücks in die spezifische Form bzw. in die spezifischen Abmessungen des gewünschten Produktes kann hierbei ein unmittelbares Ergebnis des Formprozesses sein. Insbesondere kann mit dem erfindungsgemäßen Verfahren der Rollrand bzw. die Außenfläche des Rollrandes bei Vials oder Karpulen oder bei Spritzen die Außenfläche des Spritzenkonus' geformt werden.

Eine Ausformung des Werkstückes in die Produktform kann jedoch auch durch mehrere, verschiedene Formprozesse erreicht werden. Neben den inneren und äußeren Mantelflächen des Werkstücks kann beispielsweise bei der Herstellung von Glasflaschen, -fläschchen oderbehältern auch die Hals- und Fläschchenmündungsgeometrie ausgeformt werden.

Mit der erfindungsgemäßen Vorrichtung können die Reinigungsintervalle deutlich verlängert werden. Gleichzeitig sinkt der produktionsbedingte Ausschuss. Dies wird durch die einzelnen Komponenten der Vorrichtung gewährleistet.

Die Vorrichtung ist insbesondere zum Formen eines rohrförmigen Werkstücks ausgebildet. Hier werden die inneren und äußeren Mantelflächen maßgeblich durch die Geometrie des Rohres bestimmt. In einer bevorzugten Ausführungsform der Erfindung ist das innere Formwerkzeug als Dorn ausgebildet. Eine entsprechende Vorrichtung eignet sich insbesondere zur Herstellung von Glasbehältern wie Pharma-Primärverpackungen, beispielsweise von Fläschchen, Karpulen oder Spritzen, durch Umformen von Glasrohren.

Auf Grund der Fixierung der äußeren Formgebungsrolle wird diese während des Umformvorgangs nicht durch das rotierende Werkstück angetrieben bzw. in Drehung versetzt. Zwischen der äußeren Formgebungsrolle und dem Werkstück besteht somit eine Relativbewegung. Durch diese Relativbewegung wirken Scherkräfte auf das Werkstück. Überraschenderweise hat sich herausgestellt, dass sich diese bei einer ausreichenden Schmierung der Formrolle nicht nachteilig auf die Qualität der umgeformten Werkstücke auswirkt. Vielmehr hat sich herausgestellt, dass die Scherkräfte einen positiven Effekt aufweisen. So wird das zu verformende Material durch die Scherkräfte in besonders vorteilhafter Weise zu den Formwerkzeugen geführt und an diese gedrückt, was wiederum die Ausformung des Werkstücks erleichtert.

Die Arretierung der Formrolle erfolgt mit einer lösbaren Verbindung, so dass die Formgebungsrolle zwischen den einzelnen Formgebungsprozessen gelöst und um einen Winkel $\alpha$ in der Arretierungseinrichtung gedreht werden kann. Der Winkel $\alpha$ kann gemäß einer Ausführungsform vordefiniert werden. Die Drehung der Formgebungsrolle zwischen den Heißumformungsvorgängen um den Winkel $\alpha$ führt somit dazu, dass der nachfolgende Heißumformungsvorgang durch einen benachbarten Oberflächenabschnitt der Formgebungsfläche erfolgt. Pro umgeformten Werkstück wird also nur ein geringer Anteil der Oberfläche der Formrolle eingesetzt. Durch ein Drehen der Formgebungsrolle zwischen den einzelnen Formgebungsschritten wird dabei eine gleichmäßige Abnutzung der Formgebungsfläche über die gesamte Oberfläche der Formgebungsrolle gewährleistet.

Ein weiterer Vorteil der erfindungsgemäßen Arretierungseinrichtung liegt darin, dass durch das Drehen der Formgebungsrolle zwischen den einzelnen Formgebungsprozessen in jedem Formgebungsprozess bzw. bei jedem umzuformenden Werkstück eine kalte Stelle des Formwerkzeugs verwendet wird. Somit heizt sich die Formrolle während der Produktion nicht oder nur sehr wenig auf. Dies ist besonders vorteilhaft, da somit die Rußbildung durch Verbrennung bzw. Pyrolyse des Schmieröls erheblich verringert wird. Zudem verringert sich auch der Verschleiß des Formwerkzeugs, so dass die Lebenszeit der Formwerkzeugs verlängert werden kann.

Die Vorrichtung weist eine Vorrichtung zum Aufbringen des Schmieröls auf die Formgebungsfläche der Formgebungsrolle auf. Hierbei wird das Schmieröl pro Applikationsvorgang stets nur auf einem Teilbereich der Formgebungsfläche aufgebracht. Bevorzugt erfolgt bei jedem Takt ein Aufbringen von Schmieröl auf einen Teil der Formgebungsrolle.

Eine Ausführungsform sieht vor, dass die Vorrichtung zur Abgabe von Schmieröl fest in der Vorrichtung installiert sein, d.h. der Abstand von Sprühdüse zur Oberfläche des zu besprühenden Formwerkzeugs ist konstant. Hier kann die beispielsweise Sprühdüse in die Formstation oder ein äußeres Formwerkzeug integriert sein. Auch eine Anbringung unterhalb eines äußeren Formwerkzeuge ist möglich.

Gemäß einer Ausführungsform erfolgt die Applikation des Schmieröls durch einen Tropföler. Als besonders vorteilhaft haben sich Tropföler mit einer automatischen, regelmäßigen Ölabgabe herausgestellt.

Bevorzugt ist die Vorrichtung zum Aufbringen des Schmieröls in der Vorrichtung so positioniert, dass die Auslassöffnung der Vorrichtung zum Aufbringen des Schmieröls und das Formwerkzeug in einem Winkelabstand von zumindest 45° um die Rotationsachse des Werkstücks herum angeordnet sind. Somit ist nur wenig Schmieröl in Kontakt mit dem heißen Glas. Vorzugsweise liegt der Winkel $\beta$ im Bereich von 90 bis 270°, besonders bevorzugt im Bereich von 160 bis 200°, d.h. die Vorrichtung zum Aufbringen des Schmieröls ist in der Vorrichtung auf der gegenüberliegenden Seite zum Glaseingriff angeordnet.

Gemäß einer Ausführungsform liegt die abgegebene Ölmenge pro Auftrag im Bereich von 0.01 bis 0.1 g, besonders bevorzugt im Bereich von 0.03 bis 0.05 g. Durch die geringe Menge an appliziertem Schmiermittel kann die Kontamination des Produktes, der Formwerkzeuge sowie der Produktionsumgebung maßgeblich verringert werden. Gleichzeitig wird jedoch bei der erfindungsgemäßen Vorrichtung eine ausreichende Schmierwirkung erreicht.

Als Schmiermittel können bei der erfindungsgemäßen Vorrichtung beliebige Öle mit einer Viskosität < 600 mm$^2$/s und einem Flammpunkt und/oder Pyrolysepunkt > 200°C, bevorzugt > 250 °C eingesetzt werden. Somit können hier die gleichen Öle wie bei den bekannten Standardprozessen eingesetzt werden.

Die Vorrichtung ist insbesondere zum Formen eines rohrförmigen Werkstücks ausgebildet. Hier werden die inneren und äußeren Mantelflächen maßgeblich durch die Geometrie des Rohres bestimmt. In einer bevorzugten Ausführungsform der Erfindung ist das innere Formwerkzeug als Dorn ausgebildet. Eine entsprechende Vorrichtung eignet sich insbesondere zur Herstellung von Glasbehältern wie Pharma-Primärverpackungen, beispielsweise von Fläschchen, Karpulen oder Spritzen, durch Umformen von Glasrohren.

Die Formgebungsrolle weist eine rotationssymmetrische Querschnittsfläche auf. Gemäß einer Ausführungsform der Erfindung weist die Formgebungsrolle in einer Mittelebene senkrecht zur Drehachse der Formgebungsrolle einen zylindrischen Querschnitt auf. In dieser Ausführungsform ist die Formgebungsrolle somit rund. Dies hat den Vorteil, dass in Schritt c) der Winkel, um den die Formgebungsrolle gedreht wird, frei gewählt werden kann bzw. eine genaue Einstellung des Drehwinkels $\alpha$ nicht notwendig ist.

Alternativ weist die Formgebungsrolle in einer Mittelebene senkrecht zur Drehachse der Formgebungsrolle einen polygonen Querschnitt mit mehreren Formgebungsflächen auf. Bei Formgebungsrollen mit polygonem Querschnitt ist der in Schritt c) einzuhaltende Drehwinkel $\alpha$ abhängig von der Anzahl der Formgebungsflächen. Vorzugsweise weist die Formgebungsrolle 6 bis 18 Formgebungsflächen auf.

In einer Weiterbildung dieser Ausführungsform sind die Kanten der Formgebungsflächen eben, konvex oder konkav geformt.

Durch das erfindungsgemäße Drehen der Formgebungsrolle nach jedem Formgebungsprozess wird bei jedem umzuformenden Werkstück eine kalte Stelle des Formwerkzeugs verwendet und die Formgebungsrolle heizt sich insgesamt während der Produktion nur wenig auf. Gemäß einer Weiterbildung der Erfindung weist die Vorrichtung eine zusätzliche Einrichtung zur Kühlung der Formgebungsrolle auf. Die Kühlung kann hierbei durch aktive oder passive Wärmeleitung erfolgen.

Eine Ausführungsform der Erfindung sieht vor, dass die Vorrichtung einen Kühlkörper aufweist, der direkt oder indirekt mit der Vorrichtung zur Aufnahme der Formrolle verbunden ist. Die Formrolle in thermischen Kontakt zu einem Kühlkörper steht, wobei der Kühlkörper bevorzugt ein Mittel für eine interne Kühlung aufweist, so dass Prozesswärme abgeführt werden kann. Das Kühlmittel ist hierbei nur indirekt über den Kühlkörper mit der Formrolle in Kontakt. Alternativ oder zusätzlich weist die Vorrichtung eine Luftkühlung zur Kühlung der Formrolle auf.

Bevorzugt ist die Vorrichtung derart ausgestaltet, dass durch eine Wärmeableitung die Temperatur an der Oberfläche des Formwerkzeugs höchstens 250°C, bevorzugt höchstens 180°C und besonders bevorzugt höchstens 100°C beträgt. Hierbei wird insbesondere unter der Oberfläche des Formwerkzeugs der Teil des Formwerkzeugs verstanden, der während des Umformprozesses in Kontakt mit dem heißen Glas kommt. Die Oberflächentemperatur der Formrollen wird hierbei mit Hilfe von Oberflächen-Kontaktthermometer direkt neben dem Glaskontakt Punkt sporadisch gemessen. Durch die geringen Temperaturen wird nahezu kein Öl verbrannt, so dass die Rußentwicklung signifikant reduziert und auf der Formgebungsoberfläche der Formgebungsrolle keine oder nur in sehr geringem Maße Verbrennungsrückstände abgelagert werden.

Die erfindungsgemäße Vorrichtung weist somit vorzugsweise ein Reinigungsintervall von zumindest 8 h oder sogar von zumindest 12 h auf. Unter dem Reinigungsintervall wird dabei der zeitliche Abstand zwischen zwei Standzeiten der Vorrichtung zur Reinigung der Formwerkzeuge verstanden.

Des Weiteren betrifft die Erfindung ein Verfahren zum Formen eines Werkstücks aus Glas mit fixierten Formgebungsrollen. Hierbei umfasst das Verfahren zumindest die Verfahrensschritt a) bis c) mit Schritt a) Erwärmen des Werkstücks bis zum Erweichen, mit Schritt b) Ausformen der äußeren und inneren Oberflächen des Werkstücks durch zumindest ein Formwerkzeug mit einer Formgebungsrolle in zumindest einem Formungsschritt als sowie mit Schritt c) lösen der Verriegelungseinrichtung, Drehen der Formgebungsrolle um einen vordefinierten Winkel $\alpha$ und erneute Fixierung der Formgebungsrolle, so dass bei einer Wiederholung der Schritte a) bis b) ein anderer Teil der Formgebungsfläche der Formgebungsrolle in Kontakt mit dem Werkstück kommt. Bevorzugt wird das Werkstück hierbei durch ein inneres und ein äußeres Formwerkstück geformt. Bevorzugt ist das Werkstück als Rohr, insbesondere als ein Rohr mit einem runden oder elipsoiden Querschnitt ausgebildet. Insbesondere können mit dem erfindungsgemäßen Verfahren Hals- bzw. Rollrandbereiche beispielsweise von Vials, Karpulen oder Spritzen hergestellt werden.

In Schritt a) wird das Werkstück zunächst auf eine Temperatur um die Formgebungstemperatur des verwendeten Glases aufgeheizt und in Schritt b) durch Kontakt mit dem Formwerkzeug geformt. Bevorzugt wird in Schritt b) zur Ausformung ein inneres Formwerkzeug in das Werkstück eingeführt und ein äußeres Formwerkzeug auf das Werkstück aufgebracht, so dass das Werkstück geformt wird. Das innere Formwerkzeug ist hierbei bevorzugt als Dorn ausgebildet. Das äußere Formwerkzeug weist zumindest eine Formgebungsrolle mit zumindest einer Formgebungsfläche auf. Die Formgebungsrolle ist hierbei mit einer lösbaren Verriegelungseinrichtung an einer Aufnahmevorrichtung fixiert.

Das Werkstück wird auf einem inneren Formwerkzeug positioniert und führt eine Rotationsbewegung um seinen Mittelpunkt aus. Da die Formgebungsrolle des äußeren Formgebungswerkzeugs fixiert ist, dreht sich die Formgebungsrolle nicht, so dass während des Formvorgangs eine Relativbewegung zwischen der Formgebungsfläche und dem Werkstück ausgeführt wird. Zumindest der Teil der Formgebungsfläche, welcher während des Formvorgangs in Kontakt mit dem Werkstück kommt, ist mit einem Öl als Schmiermittel bedeckt, so dass ein Anhaften des Glases an der Formgebungsfläche vermieden wird.

Die Ölapplikation des Teilbereiches der Formgebungsfläche, der in Schritt b) die Kontaktfläche zum Glas bildet erfolgt während einem der Verfahrensschritte a) bis c). Hierbei wird Öl auf einen Teil der Formgebungsfläche, der zum Zeitpunkt des Aufbringens des Öls nicht in Kontakt mit dem Werkstück steht, aufgebracht. Gemäß einer Ausführungsform der Erfindung wird bei jedem Takt Schmieröl auf einen Teil der Formgebungsfläche der Formgebungsrolle aufgetragen. Als besonders vorteilhaft haben sich hierbei Tropföler, insbesondere Tropföler mit automatischer, regelmäßiger Abgabe herausgestellt. Eine Ausführungsform der Erfindung sieht vor, dass bei jedem Ölabgabevorgang 0.01 bis 0.1 g, bevorzugt 0.03 bis 0.05 g Öl auf die Formgebungsrolle aufgebracht wird.

In Schritt c) erfolgt ein Lösen der Verriegelungseinrichtung. Die Formgebungsrolle wird um einen Winkel $\alpha$ gedreht und anschließend erneut in der Verriegelungseinrichtung fixiert. Gemäß einer Ausführungsform der Erfindung wird der Winkel $\alpha$ vordefiniert, d.h. die Formgebungsrolle wird um einen vorher festgelegten Winkel $\alpha$ gedreht. Durch Schritt c) wird somit gewährleistet, dass bei einer Wiederholung der Schritte a) bis b) ein anderer Teil der Formgebungsfläche der Formgebungsrolle in Kontakt mit dem Werkstück kommt. Somit wird eine Erwärmung der gesamten Formgebungsrolle vermieden und für jeden Formgebungsvorgang steht eine kalte Formgebungsfläche zur Verfügung. Hierdurch wird die Rußentwicklung erheblich verringert.

Gemäß einer Variante des erfindungsgemäßen Verfahrens beträgt die Oberflächentemperatur der Formgebungsoberfläche, die beim Formgebungsprozess die Kontaktfläche zum Glas bildet, während des Umformprozesses, d.h. auch bei Kontakt mit dem erwärmten Werkstück, maximal 250 °C, bevorzugt maximal 180 °C und besonders bevorzugt maximal 100 °C. Hierbei kann die Formgebungsrolle während des Formgebungsprozesses gekühlt werden. Insbesondere kann von der Formgebungsrolle während des Formgebungsprozesses mittels Wärmeleitung Wärme abgeführt werden.

Gemäß einer Ausführungsform der Erfindung erfolgt die Wärmeableitung passiv durch Wärmeableitung. Hierbei steht die Formgebungsrolle in Kontakt mit einem Kühlkörper. Der Kühlkörper kann hierbei eine aktive Kühlung aufweisen. So sieht eine Weiterbildung vor, dass der Kühlkörper mit einem Kühlmedium, bevorzugt mit einer Kühlflüssigkeit durchspült wird.

Alternativ oder zusätzlich wird der Teil der Formgebungsfläche, welcher in Kontakt mit dem umzuformenden Werkstück steht, durch Einblasen eines Gasstroms, vorzugsweise durch Einblasen eines Luftstroms gekühlt.

Gemäß einer Ausführungsform werden in Schritt b) die äußeren Mantelflächen des Werkstücks mit einer Formgebungsrolle mit einer kreisförmigen Querschnittsfläche geformt. In dieser Ausführungsform wird die Formgebungsrolle in Schritt c) bevorzugt um einen Winkel a im Bereich von 2 bis 10°, besonders bevorzugt um einen Winkel a im Bereich von 3 bis 5° gedreht. Alternativ weist die Formgebungsrolle eine polygone Querschnittsfläche auf. Vorzugsweise weist die Formgebungsrolle eine polygone Querschnittsfläche mit 6 bis 18 Formgebungsflächen auf, wobei die Anzahl der Formgebungsflächen durch die Zahl der Polygonseiten festgelegt wird.

Eine Weiterbildung der Erfindung sieht vor, dass nach Schritt c) ein Schritt d) zur Reinigung der Formgebungsrolle erfolgt. Hierbei wird der Formgebungsprozess angehalten und die Formgebungsrolle während der Standzeit gereinigt. Gemäß einer Ausführungsform der Erfindung erfolgt der Reinigungsschritt frühestens nach 10000 Wiederholungen der Prozessschritte a) bis c), d.h. nach frühestens 10000 umgeformten Werkstücken. Alternativ erfolgt der Reinigungsschritt d) frühestens nach einer Laufzeit der Vorrichtung von 4 h, bevorzugt 8 h, besonders bevorzugt 15 h und ganz besonders bevorzugt 24 h. Somit kann die Dauer des Reinigungsintervalls erheblich verlängert werden. Vorzugsweise beträgt die Laufzeit zwischen zwei Reinigungsschritten d) größer als 24 h.

Gemäß einer anderen Weiterbildung der Erfindung erfolgt nach Schritt c) ein Reinigungsschritt e) zur Reinigung der Formgebungsrolle während einer Wiederholung der Schritte a) bis c) mit neuen Werkstücken. In dieser Weiterbildung erfolgt somit die Reinigung im laufenden Prozess. Eine Unterbrechung des Herstellungsprozesses ist somit nicht notwendig. Hierbei wird jeweils nur ein Teil der Formgebungsfläche, vorzugsweise der Teil der Formgebungsfläche, welche im vorangegangenen Schritt b) in Kontakt mit dem Werkstück stand, gereinigt. Vorzugsweise erfolgt nach jedem Formgebungsprozess mit den Schritten a) bis c) eine Reinigung der Formgebungsrolle.

Weiterhin betrifft die Erfindung einen hohlen Glasgegenstand, hergestellt oder herstellbar mit dem erfindungsgemäßen Verfahren. Der Glasgegenstand ist hierbei ein Behälter oder Teil eines Behälters und umfasst die Wandung des Behälters sowie eine Hals- bzw. Schulterpartie. Der hohle Glasgegenstand ist zumindest abschnittsweise zylindrisch mit einer kreisförmigen oder ovalen Querschnittsfläche.

Die äußeren Oberflächen des Behälters weisen an den Stellen, die beim Fertigungsprozess einem erhöhten Druck ausgesetzt und mittels eines äußeren sowie eines innenliegenden Formwerkzeugs, beispielsweise in Form eines Doms, ausgeformt wurden, eine charakteristische Oberflächenstruktur auf. Diese Oberflächenbereiche der äußeren Oberfläche des Behälters werden im Folgenden auch als charakteristische Oberflächenbereiche bezeichnet. Unter den charakteristischen Oberflächen sind im Sinne der Erfindung hierbei insbesondere die Oberflächenbereiche der äußeren Glasoberflächen zu verstehen, die unter Verwendung eines innenliegenden Dorns sowie äußerer Formwerkzeuge in einem Formgebungsschritt geformt wurden. Vorzugsweise handelt es sich bei dem Formgebungsschritt zur Formung der charakteristischen Oberflächenbereiche um den letzten Formgebungsschritt innerhalb des gesamten Formgebungsverfahrens zur Herstellung des entsprechenden Behälters.

Gemäß einer Ausführungsform handelt es sich bei dem Behälter um ein Vial oder eine Karpule. Hier ist unter dem charakteristischen Oberflächenbereich die Außenfläche des Rollrandes zu verstehen. Eine andere Ausführungsform der Erfindung sieht vor, dass der Behälter eine Spritze ist. In dieser Ausführungsform wird unter dem charakteristischen Oberflächenbereich die Außenfläche des Konus verstanden.

Die charakteristischen Oberflächenbereiche des Behälters hierbei eine Oberflächenstruktur mit einem Höhenrelief mit einer Anisotropie der Steigungen im Höhenprofil auf. Das Höhenprofil weist hierbei eine Tangentialsteigung und eine Axialsteigung auf.

Unter der Tangentialsteigung wird hierbei die Steigung des Höhenprofils in Tangentialrichtung verstanden, wobei als Tangentialrichtung dabei die Richtung der Oberflächenstruktur bezeichnet wird, die der Umlaufrichtung des Werkstücks während des Formprozesses entspricht. Unter der Axialsteigung wird dagegen die Steigung des Höhenprofils in Axialrichtung, d.h. in Richtung parallel zur Rotationsachse des Werkstücks verstanden.

Der Mittelwert der Tangentialsteigung, d.h. der Tangentialsteigungsmittelwert, sowie der Mittelwert der Axialsteigung, d.h. der Axialsteigungsmittelwert, wird hierbei erfindungsgemäß wie folgt aus dem Höhenprofil des charakteristischen Oberflächenbereiches bestimmt:

Auf der charakteristischen Oberfläche des Behälters werden drei Messfelder der Größe 1,0 mm x 1,0 mm derart platziert, dass die Messfelder einen tangentialen Abstand von 120° zu einander aufweisen und die Messfelder somit über den Umfang des charakteristischen Oberflächenbereichs gleichmäßig verteilt sind. Gleichzeitig wird jedes der Messfelder in Axialrichtung mittig auf dem charakteristischen Oberflächenbereich angeordnet. Auf Grund der rotationssymmetrischen bzw. zylindrischen Form des Behälters weist jeder charakteristische Oberflächenbereich in Tangentialrichtung eine zylindrische Wölbung auf. Diese zylindrische Wölbung wird nach der Messung des Höhenprofils automatisch korrigiert, was bei einer Messfeldgröße von 1,0 mm x 1,0 mm ohne weiteres möglich ist. Auch eine eventuell auftretende systematische, d.h. durch die Formgebung des Behälters bedingte, Wölbung des charakteristischen Oberflächenbereichs in Axialrichtung wird automatisch korrigiert. Somit werden Einflüsse der makroskopischen Containerform bei der Ermittlung und nachfolgend beschriebenen Auswertung des Höhenreliefs nicht berücksichtigt.

Die Höhenreliefs der einzelnen Messfelder im charakteristischen Oberflächenbereich können mit einem Weißlichtinterferometer erstellt werden. Hierbei wird innerhalb eines Messfeldes an jedem Punkt des Höhenreliefs jeweils die Steigung in Tangentialrichtung und in Axialrichtung bestimmt. Das Höhenrelief wird anschließend rechnerisch um die makroskopische Containerform in Axial- und Umfangsrichtung bereinigt. Anschließend werden die lokalen Steigungen aus der jeweiligen Richtungsableitung in Tangential- oder Axialrichtung errechnet. Hierbei fallen die absoluten Höhenwerte des Oberflächenreliefs heraus, da die Höhenunterschiede zwischen einem Tal und einem Berg durch Bereiche mit annähernd konstanter Steigung ersetzt werden. Abhängig von der jeweiligen absoluten Höhendifferenz zwischen Berg und Tal erstrecken sich diese konstanten Bereiche unterschiedlich breit über das Messfeld. Der Tangentialsteigungsmittelwert sowie der Axialsteigungsmittelwert für ein Messfeld werden somit als über das Messfeld gemittelter Wert des Betrages der lokalen Tangentialsteigung bzw. der lokalen Axialsteigung erhalten. Die entsprechenden Steigungsmittelwerte der einzelnen Messfelder werden wiederum arithmetisch gemittelt, so dass der Tangentialsteigungsmittelwert bzw. der Axialsteigungsmittelwert gemittelt über die drei Messfelder bestimmt werden. Hierbei wird im Folgenden, sofern nicht anders bezeichnet, unter dem Tangentialsteigungsmittelwert und dem Axialsteigungsmittelwert der wie oben beschrieben ermittelte Steigungsmittelwert über die drei Messfelder verstanden.

Die erfindungsgemäßen Behälter weisen hierbei charakteristische Oberflächenbereiche auf, in denen der Tangentialsteigungsmittelwert kleiner ist als der Axialsteigungsmittelwert. Dies kann damit erklärt werden, dass bei der Herstellung des Behälters mit feststehenden Formrollen, insbesondere beim erfindungsgemäßen Herstellungsverfahren, die verformbare Glasoberfläche des Werkstücks über die stehende Werkzeugfläche des äußeren Formwerkzeugs hinwegschleift, so dass der sich auf der Oberfläche des Formwerkzeugs befindliche Schmiermittel- bzw. Ölfilm von der Glasoberfläche abgerakelt bzw. abgetragen wird. Durch die Relativbewegung zwischen Glasoberfläche und Werkzeugoberfläche entstehen somit Riefenmuster, ähnlich wie bei einem Drehprozess.

Im Unterschied dazu zeigen Höhenprofile von Behältern, die mit einem aus dem Stand der Technik bekannten Verfahren mit beweglichen Formrollen hergestellt wurden, keine oder zumindest keine ausgeprägte Anisotropie bezüglich der Steigungen des Höhenprofils abhängig von der jeweiligen Richtungsableitung. Das Fehlen einer Vorzugsrichtung kann hier damit erklärt werden, dass beim aus dem Stand der Technik bekannten Verfahren die Werkzeugoberfläche unter erhöhtem Druck im Werkzeugberührungspunkt in Umlaufrichtung auf der Glasoberfläche des Werkstücks abrollt, wobei sich zwischen Werkzeug und Glasoberfläche ein Schmierölfilm unterschiedlicher Dicke befindet. Dieser Dickenunterschied des Schmierölfilms wird während der Abrollbewegung stempelartig auf die noch verformbare Glasoberfläche eingeprägt. Hierbei besteht kein oder zumindest kein großer Unterschied darin, wie sich das Muster in Umlaufrichtung, d.h. tangential und in Richtung der Rotationsachse, also axial, ausbildet.

Mit von der Erfindung umfasst ist demnach ein hohler Glasgegenstand, welcher hergestellt oder herstellbar ist mit dem erfindungsgemäßen Verfahren, wobei der Glasgegenstand ein Behälter oder Teil eines Behältnisses mit einem Hals- oder Schulterbereich und einer Wandung ist, wobei der Glasgegenstand die Wandung und den Hals- oder Schulterbereich des Behälters umfasst, wobei die Wandung einen runden oder ovalen Querschnitt und der Behälter einen charakteristischen Oberflächenbereich auf der äußeren Glasoberfläche des hohlen Glasgegenstandes aufweist, wobei der charakteristische Oberflächenbereich eine Tangentialrichtung und eine Axialrichtung aufweist, wobei die Tangentialrichtung der Umfangsrichtung entspricht und die Axialrichtung senkrecht zur Tangentialrichtung ist,

wobei ein Höhenprofil im charakteristischen Oberflächenbereich jeweils eine Tangentialsteigung mit einem Tangentialsteigungsmittelwert und eine Axialsteigung mit einem Axialsteigungsmittelwert aufweist, wobei die Tangentialsteigung die Steigung des Höhenprofils in Tangentialrichtung und die Axialsteigung die Steigung des Höhenprofils in Axialrichtung ist,
und wobei der Tangentialsteigungsmittelwert durch Integration der Beträge der lokalen Tangentialsteigungswerte des Höhenprofils innerhalb eines Messfeldes im charakteristischen Oberflächenbereich ermittelt wird und der Axialsteigungsmittelwert durch Integration der Beträge der lokalen Axialsteigungswerte des Höhenprofils innerhalb des Messfeldes im charakteristischen Oberflächenbereich ermittelt wird.

Gemäß einer Ausführungsform der Erfindung weisen die charakteristischen Oberflächen des Behälters eine Oberflächenstruktur mit einem Höhenrelief auf, wobei für das Verhältnis zwischen dem Tangentialsteigungsmittelwert und dem Axialsteigungsmittelwert gilt:

$$\text{Tangentialsteigungsmittelwert/Axialsteigungsmittelwert} < 0{,}60.$$

Besonders bevorzugt gilt für das Verhältnis zwischen der Tangentialsteigungsmittelwert und Axialsteigungsmittelwert

Tangentialsteigungsmittelwert/Axialsteigungsmittelwert < 0,45.

Gemäß einer Weiterbildung der Erfindung weist die äußere Mantelfläche im charakteristischen Oberflächenbereich eine Rautiefe $Rz_{axial}$ gemessen in Richtung der Längsachse des Behälters auf, die größer ist als die Rautiefe $Rz_{tangential}$ gemessen in einer Höhe der Wandung quer, d.h. im Bereich von 85 bis 95°, bevorzugt bei 90°, zur Längsachse des Behälters. Bevorzugt weist dieser Abschnitt eine umlaufende Rille oder Riefe auf.

Die Tiefe der Rille oder Riefe kann durch die maximale Einzelrautiefe $Rzi_{axial}$ des betreffenden Abschnitts beschrieben werden. Die Einzelrautiefe $Rzi_{axial}$ wird dabei gemäß der Norm DIN EN ISO 4768:1990 bestimmt.

Der charakteristische Oberflächenbereich des Behälters weist in einer Weiterbildung der Erfindung zumindest 2, bevorzugt zumindest 3 umlaufende Rillen auf. Der Abstand zwischen den einzelnen Rillen kann hierbei variieren.

Gemäß einer Ausführungsform ist der hohle Glasgegenstand eine Flasche, ein Fläschchen, Teil einer Spritze oder Teil einer Karpule. Insbesondere ist der hohle Glasgegenstand Teil eines pharmazeutischen Primärpackmittels, beispielsweise ein Pharmafläschchen.

Mit umfasst von der Erfindung ist ein hohler Glasgegenstand, wobei der Glasgegenstand durch Heißumformung eines Glasrohrs hergestellt wird und/oder entlang seiner Höhe einen rohrförmigen Abschnitt mit einer konstanten Wandstärke aufweist, in welchem die Standardabweichung der Wanddicke kleiner als 0,05 mm ist.

Mit umfasst von der Erfindung ist ferner ein hohler Glasgegenstand wie vorstehend beschrieben, wobei der charakteristische Oberflächenbereich außerhalb des rohrförmigen Abschnittes liegt, und in einem Endbereich des Glasgegenstands liegt, wobei der Durchmesser des Glasgegenstands in dem Bereich des charakteristischen Oberflächenbereichs kleiner als in dem rohrförmigen Abschnitt ist.

Das Glas des hohlen Glasgegenstandes kann insbesondere ein Borosilikatglas sein.

Detaillierte Beschreibung

Nachfolgend wird die Erfindung an Hand von Ausführungsbeispielen und der Figuren 1 bis 17 näher erläutert.

Es zeigen:

Fig. 1 und 2 schematische Darstellungen einer aus dem Stand der Technik bekannten Vorrichtung zur Heißumformung in Seitenansicht und Aufsicht,

Fig. 3 und 4 schematische Darstellungen einer Ausführungsform der erfindungsgemäßen Vorrichtung zur Heißumformung in Seitenansicht und Aufsicht,

Fig. 5 eine schematische Darstellung einer Ausführungsform des erfindungsgemäßen Herstellungsverfahrens,

Fig. 6 und 7 schematische Darstellungen einer Ausführungsform der erfindungsgemäßen Vorrichtung mit einer äußeren Formrolle mit einem polygonalen Querschnitt in Seitenansicht und Aufsicht,

Fig. 8 und Fig. 9 schematische Darstellungen eines erfindungsgemäßen Glasfläschchens,

Fig. 10 die Darstellung eines 2D-Höhenreliefs der Oberflächenstruktur eines charakteristischen Oberflächenbereichs eines aus dem Stand der Technik bekannten Behälters,

Fig. 11 die Darstellung eines 2D-Höhenreliefs der Oberflächenstruktur eines charakteristischen Oberflächenbereichs einer Ausführungsform,

Fig. 12 die die Darstellung eines 3D-Höhenreliefs der Oberflächenstruktur eines charakteristischen Oberflächenbereichs eines aus dem Stand der Technik bekannten Behälters,

Fig. 13 die Darstellung eines 3D-Höhenreliefs der Oberflächenstruktur eines charakteristischen Oberflächenbereichs einer Ausführungsform,

Fig. 14 und 15 die graphischen Darstellungen der Steigungsanalyse eines charakteristischen Oberflächenbereichs eines aus dem Stand der Technik bekannten Behälters in tangentialer und axialer Richtung und

Fig. 16 und 17 die graphische Darstellungen der Steigungsanalyse eines charakteristischen Oberflächenbereichs eines Ausführungsbeispiels in tangentialer und axialer Richtung.

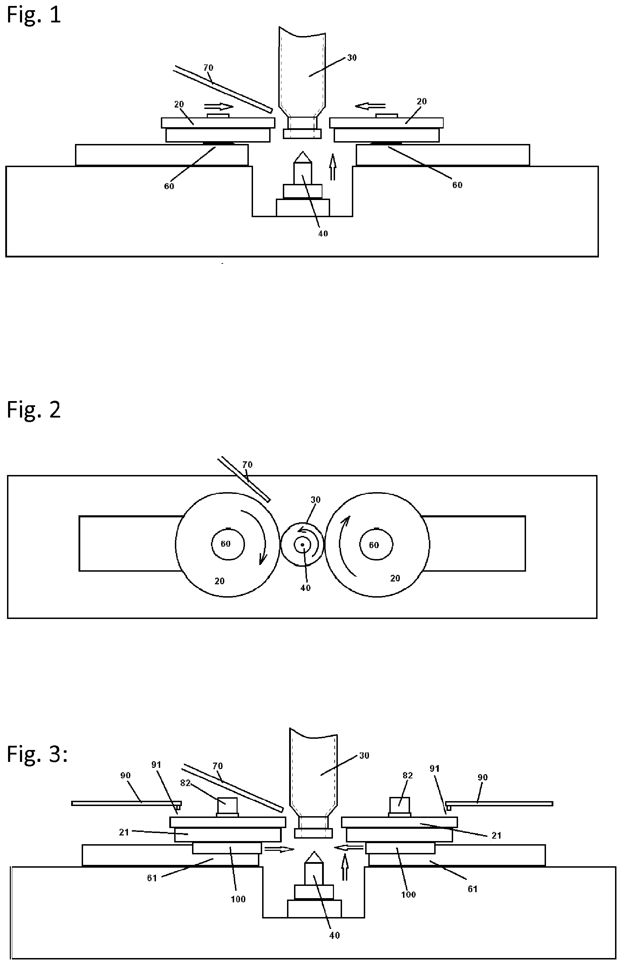

In den Figuren 1 und 2 wird eine aus dem Stand der Technik bekannte Vorrichtung zur Heißumformung 1 schematisch dargestellt. Fig. 1 zeigt hierbei die Seitenansicht und Fig. 2 eine Aufsicht. Die Vorrichtung weist hierbei ein inneres Formwerkzeug 40 auf, welches die innere Mantelfläche des Glasfläschchens ausbildet. Die äußeren Formrollen 20 sind in der Aufhängung 60 drehbar gelagert. Des Weiteren weist die Vorrichtung eine Luftkühlung 70 zur Kühlung des Glasrohres 30 auf. Während des Formprozesses erfährt das Glasrohr 30 eine Rotationsbewegung. Die Formwerkzeuge 40 und 20 bewegen sich zum zu formenden Glasrohrrohling. Da die äußeren Formwerkzeuge 20 drehbar gelagert sind, werden diese durch die Rotationsbewegung des Glasrohrs 30 ebenfalls in Drehung versetzt. Somit weisen äußere Formrolle 20 und Glasrohr 30 keine Relativbewegung zueinander auf. Des Weiteren tritt bei der Formung der äußeren Mantelfläche des Glasrohrs 30 die gesamte Mantelfläche der äußeren Formrolle 20 in Kontakt mit dem heißen Glasrohr 30. Die Kühlung des Glasrohres erfolgt mit Hilfe einer Luftkühlung 70.

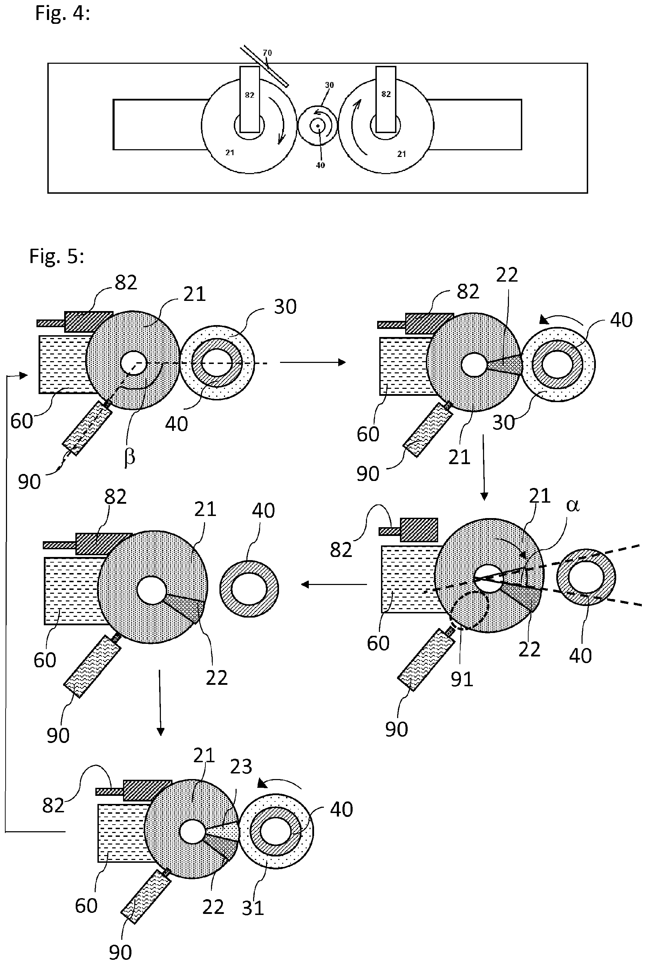

Die Figuren 3 und 4 zeigen schematische Darstellungen einer Ausführungsform einer erfindungsgemäßen Vorrichtung 2 zur Heißumformung. Fig. 3 zeigt hierbei die Seitenansicht, Fig. 4 die Aufsicht auf die Vorrichtung. Die Vorrichtung weist ein inneres Formwerkzeug 40 zur Formung der inneren Mantelflächen sowie zwei äußere Formwerkzeuge 21 zur Formung der äußeren Mantelflächen des Glasrohrs 30 auf. Das Glasrohr 30 rotiert, die Rotationsbewegung wird durch den Pfeil symbolisiert.

Die äußeren Formwerkzeuge 21 sind als Formrollen mit einer runden Querschnittsfläche ausgebildet und mit Hilfe der Aufhängung 61 frei drehbar gelagert. Während des Umformprozesses werden die Formrollen 21 jedoch durch die Arretiervorrichtung 82 fixiert, so dass eine Drehung der Formrollen 21 nicht möglich ist. Somit werden die Formrollen 21 durch das rotierende Glasrohr 30 nicht in Drehung versetzt, sondern bleiben durch die Vorrichtung 82 in ihrer Position fixiert. Somit weisen Glasrohr 30 und Formrolle 21 eine Relativbewegung zueinander auf. Da die Formrollen 21 während des Umformprozesses durch die Arretierung 82 fixiert sind, kommt nur ein kleiner Abschnitt der Mantelfläche der Formrollen 21 in Kontakt mit dem heißen Glasrohr 30, während die übrige Mantelfläche der Formrollen 21 nicht mit dem Glasrohr in Kontakt ist. Somit dient nur ein kleiner Teil der Mantelfläche der Formrolle 21 als Kontaktfläche mit dem Glasrohr.

Die erfindungsgemäße Vorrichtung 2 weist zudem Vorrichtungen 90 zum Aufbringen von Schmiermitteln auf die äußeren Formrollen 21 auf. Hierbei sind die Auslassöffnungen der Vorrichtungen 90 zum Aufbringen des Schmieröls und das Formwerkzeug in einem Winkelabstand von zumindest 45° um die Rotationsachse des Werkstücks herum angeordnet. Durch diese Anordnung der Vorrichtung 90 ist gewährleistet, dass das Schmiermittel nicht auf die Kontaktfläche der Formrolle 21 mit dem heißen Glasrohr 30, sondern auf einen anderen, kalten Bereich der Formrolle 21 aufgebracht wird. Die erfindungsgemäße Vorrichtung 2 weist in der gezeigten Ausführungsform zudem Vorrichtungen 100 zur Kühlung der Formrollen auf. Hierdurch kann eine thermische Zersetzung des Schmiermittels vermieden werden. Dies führt zu einer geringeren Rußbildung und somit auch zu einer geringeren Kontamination des Werkstücks.

Fig. 5 zeigt eine schematische Darstellung einer Ausführungsform des erfindungsgemäßen Umformungsverfahrens. Die Vorrichtung 2a entspricht hierbei der in den Figuren 3 und 4 dargestellten erfindungsgemäßen Vorrichtung 2. Zur besseren Übersichtlichkeit wird lediglich eine äußere Formrolle 21 abgebildet.

In Schritt a) wird ein gläsernes Werkstück 30 bereitgestellt und auf dem inneren Formwerkzeug 40 positioniert, wobei das Werkstück bis zum Erweichen des Glases erwärmt wurde. Das äußere Formwerkzeug 21 ist als Formrolle mit rundem Querschnitt ausgebildet und mit der Aufhängung 60 drehbar gelagert. Mit Hilfe der Arretierungsvorrichtung 82 erfolgt dabei eine Fixierung der Formrolle.

In Schritt b) erfolgt die Formung des Werkstücks 30. Hierzu erfährt das Werkstück 30 eine Rotationsbewegung um seinen Mittelpunkt. Die Rotationsbewegung wird durch den Pfeil symbolisiert. Die inneren Mantelflächen des Werkstücks 30 werden durch das innere Formwerkzeug 40 geformt. Die Formung der äußeren Mantelflächen des Werkstücks 30 erfolgt durch die Kontaktfläche 22 des äußeren Formwerkzeugs 21. Die Kontaktfläche 22 ist hierbei während des Formvorgangs mit einem Schmiermittel bedeckt.

Nach dem Formungsvorgang wird in Schritt c1) das Werkstück 30 aus der Vorrichtung 2a entfernt. Die Arretierungseinrichtung 82 wird gelöst und die Formgebungsrolle 21 um den vordefinierten Winkel α gedreht. Vorzugsweise entspricht der Winkel a dem Winkel der Kontaktfläche 22. Nachfolgend wird in Schritt c2) die Position der Formgebungsrolle 21 durch Verriegelung der Arretierungseinrichtung 82 fixiert.

Zudem erfolgt in Schritt c) die Applikation eines Schmiermittels durch die Vorrichtung 90 auf einem Teilbereich 91 der Mantelfläche der Formgebungsrolle 21. Hierbei wird das Schmiermittel auf einem Teilbereich 91 der Formgebungsrolle 21 aufgebracht, welcher im vorangegangenen Schritt b) nicht Teil der Kontaktfläche 22 war. Somit wird gewährleistet, dass der Teilbereich 91 bei der Applikation des Schmiermittels nicht oder nicht mehr durch den Kontakt mit dem heißen Glas 30 aufgeheizt ist.

Bei Wiederholung der Schritte a) und b) mit einem neuen Werkstück 31 kommt dabei ein neuer Teilbereich 23 der Mantelfläche der Formgebungsrolle 21 in Kontakt mit dem Werkstück 31. Somit erfolgt die Umformung bei jedem Werkstück mit einem anderen Teilbereich der Mantelfläche der Formgebungsrolle 21 als Kontakt- bzw. Formgebungsfläche; die jeweilige Formgebungsfläche wurde somit nicht durch einen vorherigen Formgebungsprozess aufgeheizt, sondern es liegt bei jedem Formgebungsprozess eine kalte Formgebungsfläche vor. Unter einer kalten Formgebungsfläche wird dabei insbesondere eine Formgebungsfläche mit einer Oberflächentemperatur kleiner als 250 °C verstanden.

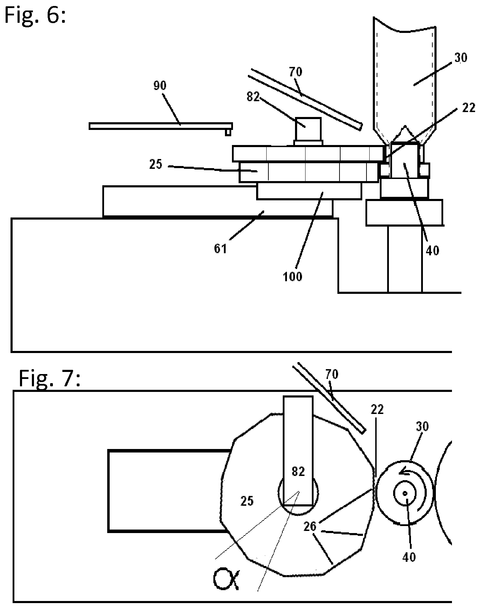

Die Figuren 6 und 7 zeigen schematische Darstellungen eines Ausführungsbeispiels mit einer Formgebungsrolle 25 mit einem polygonalen Querschnitt. Die hier gezeigte Formgebungsrolle weist dabei einen Querschnitt in Form eines Zwölfecks auf. Entsprechend sind 12 Formgebungsflächen 26 als Kontaktflächen vorhanden. Somit beträgt der in Fig. 5 gezeigte Drehwinkel $\alpha$ bei dieser Formgebungsrolle 30°.

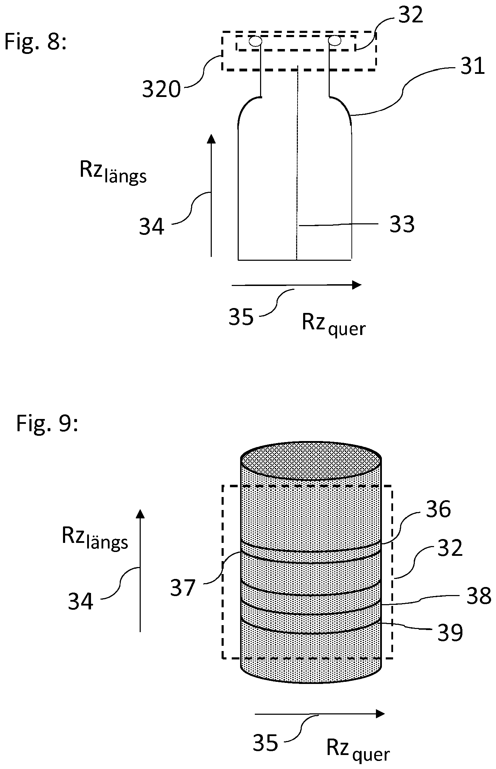

Fig. 8 zeigt eine schematische Darstellung eines umgeformten Glasfläschchens 31. Die Messrichtungen für die Tiefenrauheiten $Rz_{längs}$ und $Rz_{quer}$ bezogen auf die Längsachse 33 des Glasfläschchens im Messbereich 32 werden dabei durch die Pfeile 34 und 35 dargestellt. Hierbei kennzeichnet der Pfeil 34 die Axialrichtung und Pfeil 35 die Tangentialrichtung. Der Messbereich 32 liegt hierbei im charakteristischen Oberflächenbereich 320. In dem in Fig. 8 gezeigten Ausführungsbeispiel entspricht der charakteristische Oberflächenbereich 320 der Außenfläche des Rollrandes.

Der Messbereich 32 wird schematisch in Fig. 9 dargestellt. Die charakteristische Oberfläche 320 des Glasfläschchens, d.h. der Rollrand weist hierbei mehrere umlaufende Riefen 36, 37, 38, 39 auf. Die Riefen bzw. Rillen 36, 37, 38 und 39 sind dabei quer zur Längsachse des Glasfläschchens ausgerichtet.

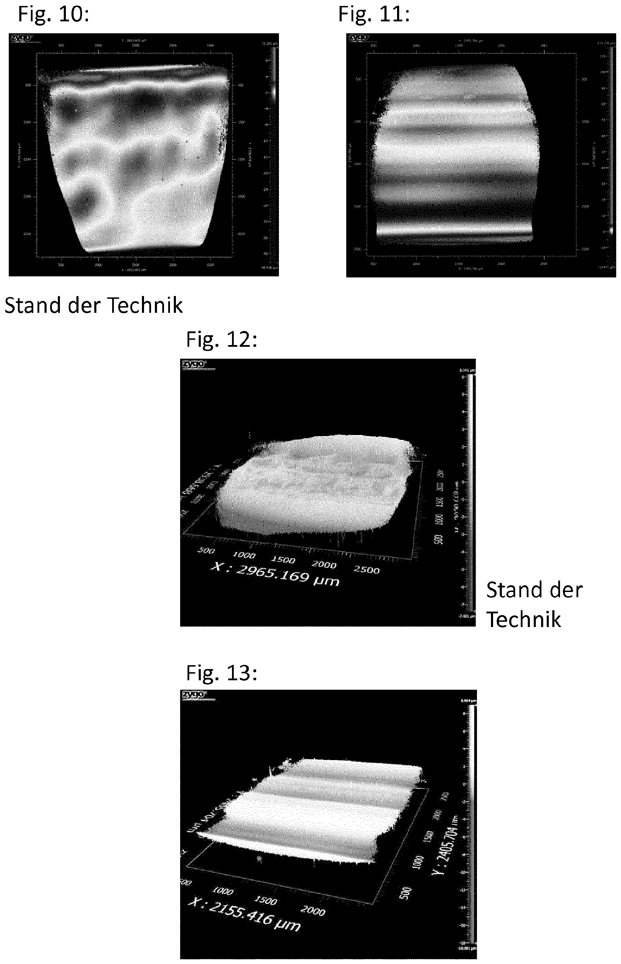

In Fig. 10 ist das 2D-Höhenprofil der Oberflächenstruktur eines charakteristischen Oberflächenbereichs eines aus dem Stand der Technik bekannten Glasfläschchens als Vergleichsbeispiel dargestellt. Das Messfeld hat hierbei eine Größe von 2 mm x 2 mm und wurde aus dem Bereich des Rollrandes entnommen. Das 2D-Höhenprofil wird hierbei in Graustufen dargestellt. Hierbei zeigt die Abszisse die Tangentialrichtung und die Ordinate die Axialrichtung an. Aus Fig. 10 wird deutlich, dass das beim Herstellungsprozess eingeprägte Muster keine Vorzugsrichtung zeigt. So ist das Muster bzw. das Höhenprofil in Umlaufrichtung, also in tangentialer Richtung ebenso ausgeprägt ist wie in Richtung der Rotationsachse, d.h. in axialer Richtung.

Fig. 11 zeigt ein 2D-Höhenprofil des charakteristischen Oberflächenbereiches eines Ausführungsbeispiels. Auch hier hat das Messfeld eine Größe von 2 mm x 2 mm und wurde aus dem Bereich des Rollrandes entnommen. Das 2D-Höhenprofil wird hierbei in Graustufen dargestellt. Hierbei zeigt die Abszisse die Tangentialrichtung und die Ordinate die Axialrichtung an. Anders als bei dem in Fig. 10 gezeigten Höhenprofil weist das Höhenprofil des Ausführungsbeispiels eine anisotrope Verteilung auf. Hierbei weisen Abschnitte mit gleichem Axialwert gleiche oder nahezu gleiche Höhen auf, während sich die Reliefhöhen der Messpunkte mit gleichen Tangentialwerten aber unterschiedlichen Axialwerten voneinander unterscheiden.

Dies ist darauf zurückzuführen, dass beim erfindungsgemäßen Verfahren die verformbare Glasoberfläche über die stehende Werkzeugoberfläche hinweg schleift und so den Undefiniert dicken Ölfilm abrakelt, so dass Riefenmuster in Tangentialrichtung entstehen. Dies wird auch an Hand von Fig. 13 deutlich. Fig. 13 zeigt hierbei das in Graustufen dargestellte 3D-Höhenrelief der Glasoberfläche eines 2 mm x 2 mm großen Messfeldes. Die x-Achse zeigt hierbei die Tangentialrichtung und die y-Achse die Axialrichtung an. Es zeigt sich ein Schleifmuster mit Rillen bzw. Riefen in Tangentialrichtung.

Im Unterschied dazu zeigt das in Fig. 12 dargestellte 3D-Höhenprofil eines charakteristischen Bereiches des Vergleichsbeispiels. Anders als das Ausführungsbeispiel zeigt das Vergleichsbeispiel keine Vorzugsrichtung im Höhenprofil. Vielmehr besteht hier kein Unterschied darin, wie sich das eingeprägte Muster tangential bzw. axial ausbildet. Zur quantitativen Erfassung der Struktur der charakteristischen Oberflächenbereiche wurden insgesamt drei jeweils ein 1 mm x 1 mm große Messbereiche ausgewählt und mittels eines Weißlichtinterferometers des Typs Zygo NexView Nx2 ein Höhenrelief erstellt. Die einzelnen Messbereiche wiesen hierbei einen Tangentialabstand von 120° zueinander auf und waren somit über den gesamten Umfang des charakteristischen Oberflächenbereichs gleichmäßig verteilt. Des Weiteren wurden die einzelnen Messbereiche so ausgerichtet, dass sie in axialer Richtung mittig im charakteristischen Oberflächenbereich angeordnet waren.

Die Höhenreliefs wurden gemessen und rechnerisch um die zylindrische Form des Messfeldes aufgrund der makroskopischen Form des Containers bereinigt.

Aus den so erhaltenen Höhenreliefs wurde an jedem Punkt eines Höhenreliefs jeweils die lokale Steigung in Tangentialrichtung als auch in Axialrichtung bestimmt. Zur Ermittlung des jeweiligen Tangentialsteigungsmittelwerts sowie des Axialsteigungsmittelwerts des jeweiligen Messfeldes wurden die Beträge der entsprechenden lokalen Steigungswerte über das gesamte Messfeld gemittelt. Die entsprechenden Steigungsmittelwerte der einzelnen Messfelder wurden wiederum arithmetisch gemittelt, so dass der Tangentialsteigungsmittelwert bzw. der Axialsteigungsmittelwert als Mittelwert über die drei Messfelder bestimmt wurden.

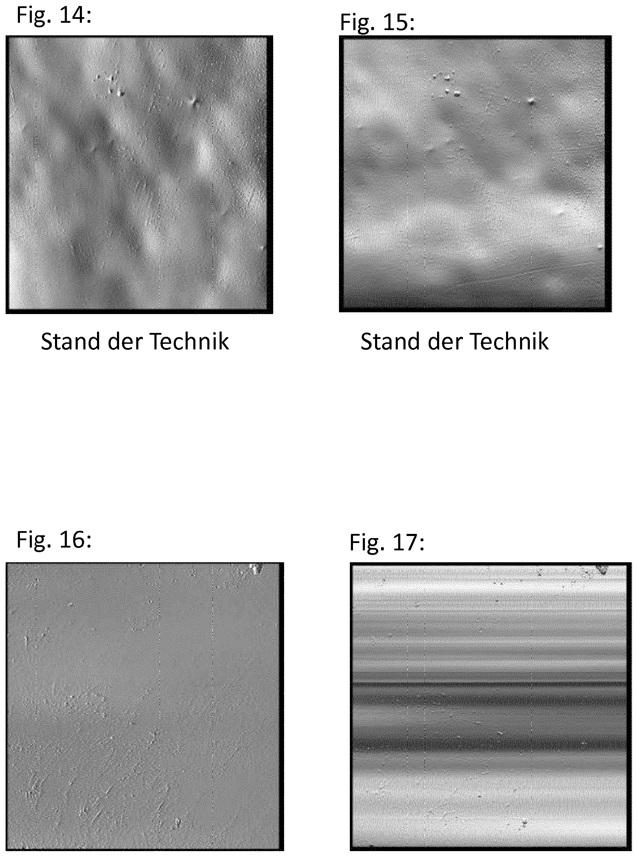

Die Figuren 14 und 15 zeigen hierbei die Steigungsanalysen in Tangentialrichtung (Fig. 14) und Axialrichtung (Fig. 15) des Vergleichsbeispiels, wobei die lokalen Steigungswerte als Graustufen dargestellt werden. Beim Vergleichsbeispiel unterscheiden sich die Werte der Tangentialsteigung und der Axialsteigung kaum voneinander, d.h. es ist keine Vorzugsrichtung bzw. Anisotropie bezüglich der Verteilung der Steigungen über das Messfeld vorhanden.

In den Figuren 16 und 17 wird die Steigungsanalyse des Ausführungsbeispiels gezeigt, wobei Fig. 16 die lokale Steigung in Tangentialrichtung und Fig. 17 die lokale Steigung in Axialrichtung zeigt. An Hand der Fig. 16 und 17 ist deutlich die Anisotropie der Steigungswerte erkennbar. Während die Steigung in Tangentialrichtung weitgehend konstant ist, variiert der Steigungswert in Axialrichtung deutlich.

Die Werte der Steigungen hängen hierbei auch vom Durchmesser der charakteristischen Oberflächen ab, die sich stets auf der Außenseite des rotationssymmetrischen Behälters befinden. Je kleiner der Durchmesser des zur Messung verwendeten Oberflächenbereichs ist, desto größer sind die auftretenden Steigungen. Dies gilt sowohl für die Steigungen in Tangentialals auch in Axialrichtung. Tabelle 1 zeigt nachfolgend Messwerte für Fläschchen mit verschiedenen Rollranddurchmessern.

Tabelle 1: Steigungsmittelwerte in Abhängigkeit von Rollranddurchmesser und Herstellungsverfahren

| | Rollranddurchmesser [mm] | Tangentialsteigungs mittelwert [μm/mm] | Axialsteigungsmittelwert [μm/mm] | Tangentialsteigungs mittelwert/ Axialsteigungsmittelwert |
|---|---|---|---|---|
| Vergleichsbeispiel 1 | 13 | 3,3 | 4,9 | 0,70 |
| Ausführungsbeispiel 1 | 13 | 1,6 | 9,1 | 0,20 |
| Vergleichsbeispiel 2 | 20 | 2,4 | 3,4 | 0,69 |
| Ausführungsbeispiel 2 | 20 | 0,9 | 4,9 | 0,13 |

Die in Tabelle 1 angegebenen Steigungswerte stellen die arithmetischen Mittelwerte mehrerer Proben dar, wobei die Werte der einzelnen Proben durch arithmetische Mittelung von Messungen an jeweils drei Messfeldern auf der Außenfläche eines Rollrandes mit einer Messfeldgröße von 1 mm x 1 mm bestimmt wurden. Hierbei wurden die Messfelder mittig in Axialrichtung platziert. Die drei Messfelder einer Messung wurden dabei in einem tangentialen Abstand von 120° zueinander platziert. Für die drei Messfelder einer Probe wurde jeweils eine Steigungsanalyse sowohl in Axial- als auch in Tangentialrichtung durchgeführt und die so erhaltenen Steigungen wurden durch Integration über das jeweilige Messfeld gemittelt. Die Vergleichsbeispiele wurden einem aus dem Stand der Technik bekannten Verfahren mit beweglichen äußeren Formrollen hergestellt, die Herstellung der Ausführungsbeispiele erfolgte mit dem erfindungsgemäßen Verfahren.

Alle Proben wurden mit Formrollen hergestellt, deren Kontaktoberflächen eine mittlere Rauheit Ra von 1,6 $\mu$m aufwiesen. Dies ist die feinste Bearbeitungsstufe, die mit bloßen Abdrehen erreicht werden kann.

Aus Tabelle 1 wird deutlich, dass die Werte der Steigungsmittelwerte sowohl bei den Ausführungs- als auch bei den Vergleichsbeispielen mit abnehmendem Radius des Rollrandes steigen.

Im Unterschied zu den beiden Vergleichsbeispielen weisen hierbei die Ausführungsbeispiele eine Anisotropie bezüglich der Steigungsmittelwerte auf. So sind die Steigungsmittelwerte in Tangentialrichtung deutlich geringer als in Axialrichtung. Entsprechend weisen die Ausführungsbeispiele auch wesentlich geringere Werte für das Verhältnis des Tangentialsteigungsmittelwertes zum Axialsteigungsmittelwertes auf als die Vergleichsbeispiele.

**Patentansprüche**

1. Vorrichtung zur Herstellung eines Werkstücks aus Glas, umfassend zumindest eine

   - Einrichtung zur Erwärmung des Glases bis zur Erweichung,
   - ein inneres Formwerkzeug zur Formung der inneren Mantelflächen des Werkstücks
   - zumindest ein äußeres Formwerkzeug zur Formung der äußeren Mantelflächen des Werkstücks umfassend eine Formgebungsrolle mit einer Formgebungsfläche, eine Vorrichtung zur Aufnahme der Formgebungsrolle und
   - einer Vorrichtung zum Aufbringen eines Schmieröls auf die Formgebungsfläche der Formgebungsrolle mit einer Auslassöffnung zur Abgabe des Schmieröls,

   wobei
   die Formgebungsrolle in der Vorrichtung zur Aufnahme frei drehbar gelagert ist und durch eine verriegelbare Arretierungseinrichtung während des Formgebungsprozesses fixierbar ist, wobei die Arretierung der Formrolle mit einer lösbaren Verbindung erfolgt, so dass die Formgebungsrolle zwischen den einzelnen Formgebungsprozessen gelöst und um einen Winkel $\alpha$ in der Arretierungseinrichtung gedreht werden kann.

2. Vorrichtung gemäß dem vorstehenden Anspruch, wobei die Vorrichtung zum Formen eines rohrförmigen Werkstücks ausgebildet ist.

3. Vorrichtung gemäß einem der vorstehenden Ansprüche, wobei die Formgebungsrolle einen runden Querschnitt in einer Mittelebene senkrecht zur Drehachse der Formgebungsrolle, einen polygonalen Querschnitt in einer Mittelebene senkrecht zur Drehachse der Formgebungsrolle mit mehreren Formgebungsflächen aufweist und/oder die Kanten der Formgebungsflächen eben, konvex oder konkav geformt sind.

4. Vorrichtung gemäß einem der vorstehenden Ansprüche, wobei bei jedem Takt ein Aufbringen von Schmieröl auf einen Teil der Formgebungsfläche der Formrolle erfolgt und/oder die Vorrichtung zum Aufbringen des Schmieröls in der Vorrichtung so positioniert ist, wobei die Auslassöffnung der Vorrichtung zum Aufbringen des Schmieröls und das Formwerkzeug in einem Winkelabstand von zumindest 45° um die Rotationsachse des Werkstücks herum angeordnet sind

5. Vorrichtung gemäß einem der vorstehenden Ansprüche, wobei die Vorrichtung einen Kühlkörper aufweist, der direkt oder indirekt mit der Vorrichtung zur Aufnahme der Formrolle verbunden ist und die Formrolle in thermischen Kontakt zu einem Kühlkörper steht, wobei der Kühlkörper bevorzugt ein Mittel für eine interne Kühlung aufweist, so dass Prozesswärme abgeführt werden kann.

6. Verfahren zum Formen von Glas umfassend zumindest die folgenden Verfahrensschritte a) bis c):

a) Bereitstellen und Erwärmen eines Werkstücks aus Glas bis zum Erweichen des Glases,
b) Formen des Werkstücks mit einem Formwerkzeug, insbesondere mit einer Vorrichtung zum Formen von Glas gemäß einem der vorstehenden Ansprüche, in einem Formungsschritt, wobei

- das Werkstück auf einem inneren Formwerkzeug positioniert wird und eine Rotationsbewegung um seinen Mittelpunkt aufweist, wobei
- die äußeren Mantelflächen des Werkstücks durch zumindest ein äußeres Formwerkzeug geformt werden, wobei das äußere Formwerkzeug zumindest eine Formgebungsrolle mit zumindest einer Formgebungsfläche aufweist und die Formgebungsrolle mit einer lösbaren Verriegelungseinrichtung an einer Aufnahmevorrichtung fixiert ist, so dass während des Formvorgangs eine Relativbewegung zwischen der Formgebungsfläche und dem Werkstück ausgeführt wird,
- zumindest der Teil der Formgebungsfläche, welcher während des Formvorgangs in Kontakt mit dem Werkstück kommt, mit einem Öl als Schmiermittel bedeckt ist,

c) Lösen der Verriegelungseinrichtung, Drehen der Formgebungsrolle um einen Winkel α und erneute Fixierung der Formgebungsrolle, so dass bei einer Wiederholung der Schritte a) bis b) ein anderer Teil der Formgebungsfläche der Formgebungsrolle in Kontakt mit dem Werkstück kommt,

wobei während einem der Verfahrensschritte a) bis c) Öl auf einen Teil der Formgebungsfläche, der zum Zeitpunkt des Aufbringens des Öls nicht in Kontakt mit dem Werkstück steht, aufgebracht wird.

7. Verfahren gemäß dem vorstehenden Anspruch, wobei in Schritt a) ein rohrförmiges Werkstück bereitgestellt wird.

8. Verfahren gemäß einem der Ansprüche 6 bis 7, wobei bei jedem Ölabgabevorgang 0.01 bis 0.1 g, bevorzugt 0.03 bis 0.05 g Öl aufgebracht werden.

9. Verfahren gemäß einem der Ansprüche 6 bis 8, wobei der Formgebungsrolle während des Formgebungsprozesses mittels Wärmeleitung Wärme abgeführt wird und/oder das äußere Formwerkzeug während des Formprozesses mit einem Kühlmedium, bevorzugt mit einer Kühlflüssigkeit, besonders bevorzugt mit Wasser durchspült wird.

10. Verfahren gemäß einem der Ansprüche 6 bis 9, wobei der Teil der Formgebungsfläche, welcher während des Formprozesses in Kontakt mit dem Werkstück steht, mit einem Gasstrom, bevorzugt mit einem Luftstrom gekühlt wird und/oder, wobei die Formgebungsfläche der Formgebungsrolle während des Formprozesses eine Temperatur von maximal 250°, bevorzugt von maximal 180°C und besonders bevorzugt von maximal 100 °C aufweist.

11. Verfahren gemäß einem der Ansprüche 6 bis 10, wobei in Schritt c) die Formgebungsrolle um einen Winkel α im Bereich von 1 bis 60°, bevorzugt um einen Winkel a im Bereich von 3 bis 10° gedreht wird.

12. Hohler Glasgegenstand, hergestellt oder herstellbar mit einem Verfahren gemäß einem der Ansprüche 6 bis 11, wobei der Glasgegenstand ein Behälter oder Teil eines Behältnisses mit einem Hals- oder Schulterbereich und einer Wandung ist, wobei der Glasgegenstand die Wandung und den Hals- oder Schulterbereich des Behälters umfasst, wobei die Wandung einen runden oder ovalen Querschnitt und der Behälter einen charakteristischen Oberflächenbereich auf der äußeren Glasoberfläche des hohlen Glasgegenstandes aufweist, wobei der charakteristische Oberflächenbereich ein Höhenprofil mit einer Tangentialsteigung und einer Axialsteigung aufweist, unter der Tangentialsteigung die Steigung des Höhenprofils in Tangentialrichtung und unter der Axialsteigung die Steigung des Höhenprofils in Axialrichtung verstanden wird und wobei für das Verhältnis zwischen dem Tangentialsteigungsmittelwert und dem Axialsteigungsmittelwert in dem charakteristischen Oberflächenbereich gilt:

Tangentialsteigungsmittelwert/Axialsteigungsmittelwert < 0,6.

13. Hohler Glasgegenstand gemäß Anspruch 12, wobei für das Verhältnis zwischen dem Tangentialsteigungsmittelwert und Axialsteigungsmittelwert in dem charakteristischen Oberflächenbereich gilt:
Tangentialsteigungsmittelwert/Axialsteigungsmittelwert < 0,45 und/oder wobei das Höhenprofil im charakteristischen Oberflächenbereich eine Anisotropie aufweist.

14. Hohler Glasgegenstand gemäß einem der Ansprüche 12 bis 13, wobei der charakteristische Bereich Abschnitte mit erhöhter Mittenrauheit $Rz_{axial}$ gemessen parallel zur Längsachse des Glasgegenstandes aufweist und wobei die Abschnitte mit erhöhter Mittenrauheit $Rz_{axial}$ sich über den gesamten Umfang des charakteristischen Bereichs erstrecken und wobei bevorzugt zumindest ein Abschnitt mit erhöhter Mittenrauheit $Rz_{axial}$ eine umlaufende Riefe oder Rille aufweist.

**15.** Hohler Glasgegenstand gemäß einem der Ansprüche 12 bis 14, wobei der Glasgegenstand eine Flasche, ein Fläschchen, bevorzugt ein Pharmafläschchen, Teil einer Karpule ist und der charakteristische Bereich durch die Außenfläche des Rollrandes gebildet wird oder der Glasgegenstand eine Spritze ist und der charakteristische Bereich durch die Außenfläche des Konus gebildet wird.

**16.** Hohler Glasgegenstand gemäß einem der Ansprüche 12 bis 15, wobei der Glasgegenstand durch Heißumformung eines Glasrohrs hergestellt wird und/oder entlang seiner Höhe einen rohrförmigen Abschnitt mit einer konstanten Wandstärke aufweist, in welchem die Standardabweichung der Wanddicke kleiner als 0,05 mm ist.

**Claims**

**1.** An apparatus for producing a workpiece made of glass, comprising at least

- a device for heating the glass until it softens;
- an inner forming tool for shaping the inner circumferential surfaces of the workpiece;
- at least one outer forming tool for shaping the outer circumferential surfaces of the workpiece, comprising a forming roller with a forming surface, an accommodation device for the forming roller; and
- a device for applying a lubricating oil to the forming surface of the forming roller, having an outlet opening for dispensing the lubricating oil;

wherein
the forming roller is mounted for free rotation in the accommodation device and can be fixed during the forming process by a lockable locking means, wherein the locking of the forming roller is effected by a releasable connection so that the forming roller can be released between the individual forming processes and can be rotated about an angle $\alpha$ in the locking means.

**2.** The apparatus according to the preceding claim, wherein the apparatus is configured for forming a tubular workpiece.

**3.** The apparatus according to any one of the preceding claims, wherein the forming roller has a round cross-sectional shape in a central plane perpendicular to the axis of rotation of the forming roller, a polygonal cross-sectional shape in a central plane perpendicular to the axis of rotation of the forming roller comprising a plurality of forming surfaces, and/or wherein the edges of the forming surfaces are flat, convex or concave.

**4.** The apparatus according to any one of the preceding claims, wherein lubricating oil is applied to a portion of the forming surface of the forming roller in each cycle; and/or
wherein the device for applying lubricating oil is oriented within the apparatus such that the outlet opening of the device for applying lubricating oil and the forming tool are arranged at an angular spacing of at least 45° about the axis of rotation of the workpiece.

**5.** The apparatus according to any one of the preceding claims, wherein the apparatus comprises a heat sink which is connected directly or indirectly to the forming roller accommodation device, and wherein the forming roller is in thermal contact with a heat sink, wherein the heat sink preferably includes a means for internal cooling so that process heat can be dissipated.

**6.** A method for forming glass, comprising at least the following method steps a) to c):

a) providing and heating a workpiece made of glass until the glass softens;
b) forming the workpiece using a forming tool, in particular using an apparatus for forming glass according to any one of the preceding claim, in a forming step in which

- the workpiece is positioned on an inner forming tool and exhibits a rotational movement around its centre; wherein
- the outer circumferential surfaces of the workpiece are shaped by at least one outer forming tool, the outer forming tool comprising at least one forming roller with at least one forming surface, and wherein the forming roller is fixed on an accommodation device by a releasable locking means, so that a relative movement is performed between the forming surface and the workpiece during the forming process;
- at least that portion of the forming surface which comes into contact with the workpiece during the forming process is covered with an oil as a lubricant;

c) releasing the locking means, rotating the forming roller about an angle $\alpha$ and re-fixing the forming roller, so that a different portion of the forming surface of the forming roller comes into contact with the workpiece when steps a) to b) are repeated;

wherein, during one of method steps a) to c), oil is applied to a portion of the forming surface which is not in contact with the workpiece at the time the oil is applied.

7. The method according to the preceding claim, wherein a tubular workpiece is provided in step a).

8. The method according to any one of claims 6 to 7, wherein 0.01 to 0.1 g, preferably 0.03 to 0.05 g of oil are applied in each oil dispensing event.

9. The method according to any one of claims 6 to 8, wherein heat is dissipated from the forming roller by thermal conduction during the forming process; and/or wherein the outer forming tool is flushed by a cooling medium during the forming process, preferably a cooling liquid, most preferably water.

10. The method according to any one of claims 6 to 9, wherein the portion of the forming surface which is in contact with the workpiece during the forming process is cooled by a gas flow, preferably by an air flow; and/or wherein, during the forming process, the forming surface of the forming roller has a temperature of not more than 250 °C, preferably not more than 180 °C, and most preferably not more than 100 °C.

11. The method according to any one of claims 6 to 10, wherein in step c) the forming roller is rotated about an angle $\alpha$ in a range from 1 to 60°, preferably by an angle $\alpha$ in the range from 3 to 10°.

12. A hollow glass article, produced or producible by a method according to any one of claims 6 to 11, wherein the glass article is a container or a portion of a container having a neck or shoulder portion and a wall, wherein the glass article comprises the wall and the neck or shoulder portion of the container, wherein the wall has a circular or oval cross-sectional shape and the container has a characteristic surface area on the outer glass surface of the hollow glass article, wherein the characteristic surface area has a height profile with a tangential slope and an axial slope, tangential slope referring to the slope of the height profile in tangential direction and axial slope referring to the slope of the height profile in axial direction, and wherein within the characteristic surface area, the following applies for the ratio between the mean tangential slope value and the mean axial slope value: mean tangential slope value / mean axial slope value < 0.6.

13. The hollow glass article according to claim 12, wherein within the characteristic surface area the following applies to the ratio between the mean tangential slope value and the mean axial slope value: mean tangential slope value / mean axial slope value < 0.45; and/or
wherein the height profile within the characteristic surface area exhibits anisotropy.

14. The hollow glass article according to any one of claims 12 to 13, wherein the characteristic area has sections with increased average roughness $Rz_{axial}$ as measured parallel to the longitudinal axis of the glass article, and wherein the sections with increased average roughness $Rz_{axial}$ extend over the entire circumference of the characteristic area, and wherein, preferably, at least one section with increased average roughness $Rz_{axial}$ includes a circumferential flute or groove.

15. The hollow glass article according to any one of claims 12 to 14, wherein the glass article is a bottle, a vial, preferably a pharmaceutical vial, part of a carpule, and the characteristic area is defined by the outer surface of the rolled edge; or wherein the glass article is a syringe and the characteristic area is defined by the outer surface of the cone.

16. The hollow glass article according to any one of claims 12 to 15, wherein the glass article is produced by hot forming of a glass tube and/or has a tubular section with a constant wall thickness along its height, with a standard deviation of the wall thickness of less than 0.05 mm.

**Revendications**

1. Dispositif de fabrication d'une pièce en verre, comprenant au moins

   - un équipement pour chauffer le verre jusqu'à ramollissement,
   - un outil de formage interne destiné à la mise en forme des surfaces d'enveloppe internes de la pièce,
   - au moins un outil de formage externe destiné à la mise en forme des surfaces d'enveloppe externes de la pièce, comprenant un cylindre de formage doté d'une surface de formage, un dispositif de support du cylindre de formage et
   - un dispositif destiné à appliquer de l'huile lubrifiante sur la surface de formage du cylindre de formage, comportant un orifice de sortie pour délivrer l'huile lubrifiante,

   dans lequel
   le cylindre de formage est monté de manière à pouvoir tourner librement dans le dispositif de support et peut être fixé par un dispositif de blocage verrouillable pendant le processus de formage, le verrouillage du cylindre de formage s'effectuant par le biais d'une liaison libérable, de sorte que le cylindre de formage peut être dégagé entre les différents processus de formage et tourné d'un angle α dans le dispositif de blocage.

2. Dispositif selon la revendication précédente, dans lequel le dispositif est conçu pour la mise en forme d'une pièce tubulaire.

3. Dispositif selon l'une des revendications précédentes, dans lequel le cylindre de formage présente une section transversale ronde dans un plan médian perpendiculaire à l'axe de rotation du cylindre de formage, une section transversale polygonale dans un plan médian perpendiculaire à l'axe de rotation du cylindre de formage, avec plusieurs surfaces de formage, et/ou les arêtes des surfaces de formage ont une forme plane, convexe ou concave.

4. Dispositif selon l'une des revendications précédentes, dans lequel, lors de chaque cycle, une application d'huile lubrifiante est effectuée sur une partie de la surface de formage du cylindre de formage et/ou le dispositif d'application d'huile lubrifiante est positionné dans le dispositif de cette manière, l'orifice de sortie du dispositif d'application d'huile lubrifiante et l'outil de formage étant installés avec un écart angulaire d'au moins 45° autour de l'axe de rotation de la pièce.

5. Dispositif selon l'une des revendications précédentes, dans lequel le dispositif présente un corps de refroidissement qui est relié directement ou indirectement au dispositif de support du cylindre de formage, et le cylindre de formage est en contact thermique avec un corps de refroidissement, le corps de refroidissement comportant de préférence un moyen de refroidissement interne, de sorte que la chaleur du processus peut être évacuée.

6. Procédé de mis en forme de verre, comprenant au moins les étapes de procédé a) à c) suivantes :

   a) mise à disposition et chauffage d'une pièce en verre, jusqu'à ramollissement du verre,
   b) mise en forme de la pièce avec un outil de formage, en particulier avec un dispositif de formage de verre selon l'une des revendications précédentes, au cours d'une étape de mise en forme, sachant que

   - la pièce est disposée sur un outil de formage interne et présente un mouvement de rotation autour de son centre, où
   - les surfaces enveloppes externes de la pièce sont mises en forme par au moins un outil de formage externe, l'outil de formage externe présentant au moins un cylindre de formage doté d'au moins une surface de formage, et le cylindre de formage étant fixé sur un dispositif de support à l'aide d'un moyen de verrouillage libérable, de sorte que pendant l'opération de mise en forme, un mouvement relatif est exécuté entre la surface de formage et la pièce,
   - au moins la partie de la surface de formage qui est en contact avec la pièce pendant l'opération de mise en forme est recouverte d'huile en tant que lubrifiant,

   c) libération du moyen de verrouillage, rotation du cylindre de formage d'un angle α et de nouveau fixation du cylindre de formage, de sorte que lors de la répétition des étapes a) à b), une autre partie de la surface de formage du cylindre de formage entre en contact avec la pièce,

   sachant qu'au cours de l'une des étapes de procédé a) à c), de l'huile est appliquée sur une partie de la surface de formage qui n'est pas en contact avec la pièce au moment de l'application de l'huile.

7. Procédé selon la revendication précédente, selon lequel une pièce tubulaire est mise à disposition à l'étape a).

8. Procédé selon l'une des revendications 6 à 7, selon lequel, lors de chaque opération de délivrance d'huile, on applique 0,01 à 0,1 g, de préférence 0,03 à 0,05 g d'huile.

9. Procédé selon l'une des revendications 6 à 8, selon lequel la chaleur est évacuée du cylindre de formage par conduction thermique pendant le processus de mise en forme et/ou l'outil de formage externe est parcouru par un fluide de refroidissement pendant le processus de mise en forme, de préférence par un liquide de refroidissement et, de manière particulièrement avantageuse, par de l'eau.

10. Procédé selon l'une des revendications 6 à 9, selon lequel la partie de la surface de formage qui est en contact avec la pièce pendant le processus de mise en forme est refroidie par un flux de gaz, de préférence un flux d'air, et/ou la surface de formage du cylindre de formage présente une température d'au maximum 250°, de préférence d'au maximum 180 °C, et de manière particulièrement avantageuse d'au maximum 100 °C pendant le processus de mise en forme.

11. Procédé selon l'une des revendications 6 à 10, selon lequel, à l'étape c), le cylindre de formage est tourné d'un angle $\alpha$ compris dans la plage allant de 1 à 60°, de préférence d'un angle a compris dans la plage de 3 à 10°.

12. Objet en verre creux, fabriqué ou pouvant être fabriqué avec un procédé selon l'une des revendications 6 à 11, l'objet en verre étant un récipient ou une partie d'un récipient comportant une zone de col ou d'épaule et une paroi, l'objet en verre comprenant la paroi et la zone de col ou d'épaule du récipient, la paroi présentant une section transversale ronde ou ovale, et le récipient présentant une zone de surface caractéristique sur la surface de verre externe de l'objet en verre creux, la zone de surface caractéristique présentant un profil de hauteur avec une pente tangentielle et une pente axiale, étant entendu que la pente tangentielle désigne la pente du profil de hauteur dans la direction tangentielle et la pente axiale désigne la pente du profil de hauteur dans la direction axiale, et le rapport entre la valeur moyenne de la pente tangentielle et la valeur moyenne de la pente axiale dans la zone de surface caractéristique étant défini comme :

$$\text{valeur moyenne de pente tangentielle/valeur moyenne de pente axiale} < 0{,}6.$$

13. Objet en verre creux selon la revendication 12, dans lequel le rapport entre la valeur moyenne de pente tangentielle et la valeur moyenne de pente axiale dans la zone de surface caractéristique est défini comme :
valeur moyenne de pente tangentielle/valeur moyenne de pente axiale < 0,45 et/ou le profil de hauteur dans la zone de surface caractéristique présentant une anisotropie.

14. Objet en verre creux selon l'une des revendications 12 à 13, dans lequel la zone caractéristique présente des portions avec une rugosité moyenne arithmétique $Rz_{axial}$ accrue, mesurée parallèlement à l'axe longitudinal de l'objet en verre, et les portions à rugosité moyenne arithmétique $Rz_{axial}$ accrue s'étendant sur tout le pourtour de la zone caractéristique, et de préférence au moins une portion à rugosité moyenne arithmétique $Rz_{axial}$ accrue présentant une cannelure ou une rainure périphérique.

15. Objet en verre creux selon l'une des revendications 12 à 14, dans lequel l'objet en verre est une bouteille, un flacon, de préférence un flacon pharmaceutique, une partie d'une carpule, et la zone caractéristique est constituée de la face externe du bord roulé, ou l'objet en verre est une seringue et la zone caractéristique est constituée de la surface externe du cône.

16. Objet en verre creux selon l'une des revendications 12 à 15, dans lequel l'objet en verre est fabriqué par formage à chaud d'un tube en verre et/ou présente, le long de sa hauteur, une portion tubulaire avec une épaisseur de paroi constante, dans laquelle l'écart type de l'épaisseur de paroi est inférieur à 0,05 mm.

## Fig. 1

## Fig. 2

## Fig. 3:

Fig. 4:

Fig. 5:

Fig. 6:

Fig. 7:

Fig. 8:

32

320

31

$Rz_{längs}$

34

33

35    $Rz_{quer}$

Fig. 9:

$Rz_{längs}$

34

37

36

32

38

39

35    $Rz_{quer}$

Fig. 10:

Fig. 11:

Stand der Technik

Fig. 12:

Stand der Technik

Fig. 13:

Fig. 14:

Stand der Technik

Fig. 15:

Stand der Technik

Fig. 16:

Fig. 17:

**EP 3 677 554 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202004004560 U1

- WO 2018011148 A1